# EUROPEAN PATENT APPLICATION

(11) **EP 2 108 639 A1**
(43) Date of publication of application: **14.10.2009**
(21) Application number: 08154144.3
(22) Date of filing: 07.04.2008
(51) Int. Cl.: C07C 29/10, C07C 31/20

(54) **Process for the preparation of monopropylene glycol**

(71) Applicant: SHELL INTERNATIONALE RESEARCH MAATSCHAPPIJ B.V., 2596 HR Den Haag (NL)
(72) Inventor: Beckers, Johannes, Gerhardus, Joseph, 1031 CM Amsterdam (NL); Van Kessel, Gerardus, Martinus, Maria, 1031 CM Amsterdam (NL)
(74) Representative: Zeestraten, Albertus W. J.

(57) **Abstract**

This invention relates to a process for the preparation of monopropylene glycol, wherein a base is added to a composition comprising propylene oxide and from 3 to 3000 ppmw of a carboxylic acid, based on total composition, forming a salt, and the propylene oxide is reacted with water in the presence of the salt to obtain monopropylene glycol.

## Description

### Field of the invention

This invention relates to a process for the preparation of monopropylene glycol.

### Background of the invention

Monopropylene glycol, with IUPAC name propane-1,2-diol, is a petrochemical commodity and is used for the production of polyester fibers, as anti-freeze and as industrial coolant. Monopropylene glycol can be prepared by reaction of propylene oxide with water (hydrolysis reaction). This reaction can be carried out at high temperatures (thermal hydrolysis) or in the presence of a catalyst (catalytic hydrolysis).

The propylene oxide used in the preparation of monopropylene glycol may be obtained by
(a) oxidation step: oxidizing ethylbenzene to yield ethylbenzene hydroperoxide, and
(b) epoxidation step: reacting propylene with the obtained ethylbenzene hydroperoxide to yield propylene oxide in the presence of a catalyst.

During said oxidation step carboxylic acids may be obtained as by-products. The presence of these carboxylic acids may decrease the catalytic activity of the catalyst used in said subsequent epoxidation step, for instance as described in US 6,646,138 B2. The process described in said US 6,646,138 B2 comprises a step between the oxidation step and the epoxidation step in order to remove the acids by extraction with an aqueous alkaline solution.

US-A 5,723,637 also describes a process wherein the presence of carboxylic acids adversely affects the catalytic activity of Ti-containing solid catalyst which is used in the reaction of propylene with a solution of ethylbenzene hydroperoxide in ethylbenzene. The ethylbenzene hydroperoxide in ethylbenzene solution is obtained by liquid phase autoxidation of ethylbenzene. The obtained solution contains carboxylic acids such as lactic acid, formic acid, acetic acid, propionic acid, benzoic acid and the like. According to said US-A 5,723,637, this solution is washed with an aqueous alkali solution to bring the lactic acid concentration to 5 ppm by weight or less.

From said US 6,646,138 B2 and US-A 5,723,637 it is evident that commercially produced propylene oxide may still contain carboxylic acids as by-products.

Further, it is possible to treat propylene oxide, after it has been made, with an aqueous base to yield pure propylene oxide before its use in the preparation of monopropylene glycol, for instance as described in US-A - 3,574,772.

In US-A 3,574,772 a process is described in which propylene oxide is doped with several contaminants, and the thus obtained crude propylene oxide is treated with an aqueous alkaline solution. A water phase, containing the contaminants, was separated from a propylene oxide phase. This process suffers the disadvantage that an extra step in the process is needed, namely the step of separating the propylene oxide from the aqueous alkaline phase before reaction of the propylene oxide with the water to produce monopropylene glycol.

US-A 2,807,651 discloses a process for the catalytic hydrolysis of propylene oxide using sulphur dioxide as catalyst. The performance of such catalyst is compared with the performance of sodium hydroxide as catalyst. Thereto about 1.1 mol NaOH was used as catalyst for 15.5 moles propylene oxide. This reaction yielded a low selectivity to monopropylene glycol. The weight ratio of monopropylene glycol to polypropylene glycol was 2.3.

An object of the present invention is to provide a process for the preparation of monopropylene glycol from propylene oxide, in which process the formation of by-products during the preparation of monopropylene glycol is reduced. An additional object is to provide such an monoproylene glycol production process that prior to the preparation of monopropylene glycol no steps need to be carried out for purifying the propylene oxide, such as treatment with an aqueous base and then separating the purified propylene oxide from the aqueous base.

### Summary of the invention

Surprisingly, it was found that the above objects are achieved if a base is added to a composition comprising propylene oxide and from 3 to 3000 ppm by weight (parts per million weight:ppmw) of a carboxylic acid, whereby a salt is formed, and the propylene oxide is reacted with water in the presence of said salt.

Accordingly, the invention relates to a process for the preparation of monopropylene glycol, wherein a base is added to a composition comprising propylene oxide and from 3 to 3000 ppmw of a carboxylic acid, based on total composition, forming a salt, and the propylene oxide is reacted with water in the presence of the salt to obtain monopropylene glycol.

It has been found that where a base is added to a composition comprising propylene oxide and from 3 to 3000 ppmw of a carboxylic acid ("crude propylene oxide"), and the propylene oxide is reacted with water, as in the present process, only a small amount of by-products is formed, despite the presence of any remaining carboxylic acid and of the salt that is formed upon reaction of the carboxylic acid with the added base. Apart from the fact that relatively pure monopropylene glycol is formed in this way, a further advantage thereof is that the present process may result in less corrosion of equipment.

### Detailed description of the invention

In the present process, the composition to which the base is added, comprises from 3 to 3000 ppmw of a carboxylic acid, based on total composition, that is to say based on the total weight of propylene oxide, carboxylic acid and optionally further contaminants. Specifically, said composition comprises 50 to 2500 ppmw, and more specifically 70 to 1500 ppmw, of carboxylic acid. The carboxylic acid may be acetic acid, formic acid, propionic acid, or a mixture of two or more thereof, and specifically comprises acetic acid.

In addition to said carboxylic acids, other contaminants in crude propylene oxide may be alkenes and alkanes, and oxygen containing compounds such as aldehydes, ketones, alcohols, ethers, esters and water, for example acetone, acetic aldehyde, propionic aldehyde, and methyl formate.

The presence of carboxylic acids, such as acetic acid, in crude propylene oxide leads to a low pH, such as suitably a pH ranging from 2 to 6.5, of a reaction system comprising such crude propylene oxide and water. This may result in the following by-products: propionaldehyde, 1,3-methylethyldioxalane, and allyl alcohol. The formation of these by-products causes propylene oxide loss.

Crude propylene oxide may be obtained by epoxidation of propene with a hydroperoxide, such as tertiary butyl hydroperoxide, ethylbenzene hydroperoxide or hydrogen peroxide.

It has been found that when reacting water with crude propylene oxide, the propylene oxide loss to propionaldehyde, 1,3-methylethyldioxalane, and allyl alcohol is significant. However, in the present process, where a base is added to said composition thereby forming a salt, the propylene oxide loss upon reaction of the propylene oxide with water is advantageously substantially reduced.

Suitable bases to be added in the present invention, include inorganic or organic basic compounds such as alkali metal, alkaline earth metal or ammonium hydroxides, carbonates or bicarbonates, or ammonia, or amines which may be primary, secondary or tertiary. Examples of such bases are sodium hydroxide, sodium bicarbonate, potassium hydroxide, potassium bicarbonate, ammonium hydroxide, calcium bicarbonate, calcium hydroxide, cesium hydroxide, N-methylmorpholine and the like. Preferably, in the present invention the base is an alkali metal or alkaline earth metal hydroxide, more preferably potassium or sodium hydroxide, most preferably sodium hydroxide.

Therefore, if in the present process, for example, sodium hydroxide is added as a base and acetic acid is present as carboxylic acid in the (crude) propylene oxide composition, sodium acetate (NaOAc) will be formed as the salt in the presence of which the reaction between propylene oxide and water takes place.

In the present process, the base can be added as a solid or as an aqueous solution. Further, the base can be added to a mixture obtained by mixing water and the composition comprising propylene oxide and carboxylic acid and/or the base can be added to said composition and/or to said water before said composition and said water are mixed.

In the present process, the concentration of the added base based on the total weight of water and the composition comprising propylene oxide and carboxylic acid is preferably from 1 to 2000 ppmw, more preferably from 10 to 1500 ppmw, more preferably from 20 to 1000 ppmw, more preferably from 50 to 500 ppmw and most preferably from 70 to 200 ppmw. Further, the added base may be added in a stoichiometric excess over the carboxylic acid in the propylene oxide composition such that all of the carboxylic acid is neutralized into a carboxylate salt. Preferably, the amount of added base may be such that the pH of the reaction mixture, comprising monopropylene oxide and water is less than 7. The pH of the reaction mixture may be from 3 to 10, more specifically 3 to 7, even more specifically 4.5 to 6. When the reaction mixture is kept at these pH values, the selectivity towards monopropylene glycol is retained sufficiently high.

In the present process the weight ratio of water to the composition comprising propylene oxide and carboxylic acid is preferably from 10:1 to 1:1, more preferably from 6:1 to 3:1.

The temperature in the present process is preferably at most 350°C, more preferably at most 320°C, more preferably at most 300°C, more preferably at most 275°C, and most preferably at most 250°C, and preferably at least 130°C, more preferably at least 140°C, more preferably at least 160°C and most preferably at least 170°C.

Preferably, the pressure in the present process is higher than atmospheric pressure. Suitable pressures are from 1000 to 7000 kPa, preferably from 4000 to 7000 kPa.

It has been found that the reaction mixture obtained in the present process contains less by-products, such as propionaldehyde, 1,3-methylethyldioxalane, and allyl alcohol, than the reaction mixture obtained using pure propylene oxide. Therefore, the present invention also relates to monopropylene glycol obtainable according to the present process, that contains allyl alcohol in an amount in the range from 0.5 to 200 ppmw.

Preferably, the reaction mixture is separated into a product stream and a recycle stream, wherein the product stream comprises monopropylene glycol and the recycle stream comprises water. Such separation can easily be carried out by someone skilled in the art. For example, the separation may comprise removing water from the reaction mixture by multi-effect distillation, for instance as described in Perry, H., Green, D., Perry's Chemical Engineers' Handbook, McGraw-Hill, Sixth Edition, 1984, p. 11-31 and 11-40. After removing the water, one further distillation leads to pure monopropylene oxide.

Distillation of the reaction mixture containing monopropylene glycol and water gives an overhead fraction containing water. This distillation can be carried out at atmospheric pressure or at a pressure which is lower or higher than atmospheric pressure. Suitable pressures are in the range from 10 to 1500 kPa (0.1 to 15 bar). Suitable temperatures are in a range from 10 to 250 °C. Preferably, where multi-effect distillation is performed, both the pressure and temperature are decreased towards the last column. The overhead fraction(s) containing water as separated during said distillation(s) can be recycled to the step of reacting crude propylene oxide with water. Recycling provides for a continuous monopropylene glycol production process. A part of the recycle stream may be discharged as a bleed in order to prevent accumulation of any low-boiling by-products.

Distillation of the monopropylene glycol containing bottom fraction originating from the distillation wherein water was removed, gives an overhead fraction containing pure monopropylene glycol. The bottom stream, containing comprising salt and the heavier glycol products including dipropylene glycol, is sent to storage. This distillation can be carried out at a pressure from 5 to 10 kPa, and at a temperature from 70 to 170 °C.

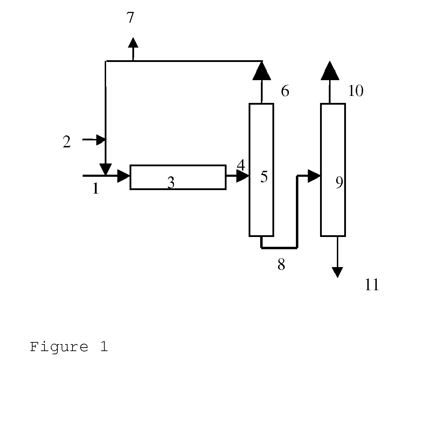

Fig. 1 is a schematic representation of one embodiment of the present process. In this process water is recycled to the reaction zone, followed by distillation to recover monopropylene glycol product. An aqueous stream 1 comprising crude propylene oxide and an aqueous stream 2 comprising a base are led into a reactor 3 where they are mixed. The reactor 3 for producing monopropylene glycol may be an adiabatic plug flow reactor, for instance as described in Perry, H., Green, D., Perry's Chemical Engineers' Handbook, McGraw-Hill, Sixth Edition, 1984, p. 4-33. In the reactor the mass ratio of water to crude propylene oxide may be 5:1 and the mass ratio of base to water may be 45 ppmw.

Crude propylene oxide is converted at a temperature of e.g. 210 °C and a pressure of e.g. 5000 kPa. The reaction mixture is led out of the reactor 3 as a stream 4 to a distillation column 5. In column 5, water is separated from the reaction mixture at a temperature, which may be 250 °C and a pressure, which may be 1300 kPa. The overhead stream 6 comprising water is recycled from column 5 to reactor 3. Optionally, a part of the recycle stream 6 is discharged from the process as a bleed stream 7. The bottom stream 8 of column 5 comprising the monopropylene glycol product is led to distillation column 9. In column 9, monopropylene glycol is distilled overhead at a temperature, which may be 170 °C and a pressure, which may be 10 kPa. The overhead stream 10 comprising pure monopropylene glycol is recovered as product. The bottom stream 11, comprising salt and the heavier glycol products including dipropylene glycol, may be further treated to recover dipropylene glycol.

The process according to the present invention is further elucidated by reference to the following examples. All reaction mixtures obtained in the examples have been analysed by gas chromatography.

### Comparative Example 1

A 1 1 batch reactor, equipped with a stirrer and a heating/cooling system, was charged with 350 mL demineralised water that had first been subjected to Millipore filtration. Oxygen was removed by stripping with nitrogen for 3 minutes. The reactor was heated in about 2 hours to 250°C. Thereafter 70 g of a propylene oxide composition, comprising 28 ppmw of carboxylic acid, comprising mainly acetic acid (HOAc), based on total weight of propylene oxide (see Table 1), was injected into the reactor wherein the nitrogen pressure was kept at 50 bar. Six minutes after having added the propylene oxide, the reaction mixture was removed from the reactor and passed through a pipe having a length of 9 meter and a diameter of 0.3175 cm which was immersed in a water/ice mixture, thereby stopping the reaction between propylene oxide and water.

The cooled reaction mixture was analyzed with gas chromatography and comprised monopropylene glycol, water and the by-products propionaldehyde, 1,3-methylethyldioxalane, and allyl alcohol. The amounts of propionaldehyde, 1,3-methylethyldioxalane, and allyl alcohol in the reaction mixture and the pH of said reaction mixture are shown in Table 2.

### Comparative Example 2

The procedure of Comparative Example 1 was repeated, with the proviso that 100 mg of acetic acid (HOAc) was added to the propylene oxide before injection of the propylene oxide into the reactor. Therefore, the total amount of carboxylic acid based on total weight of propylene oxide composition was 1457 ppmw (see Table 1), i.e. the total of 1429 ppmw of the added HOAc and 28 ppmw of the carboxylic acid already contained in the original propylene oxide composition (see Comparative Example 1).

From Table 2 it appears that by reacting water with propylene oxide containing more carboxylic acid, more by-products are formed. The amounts of each of propionaldehyde, 1,3-methylethyldioxalane, and allyl alcohol, and the total amount of propionaldehyde, 1,3-methylethyldioxalane, and allyl alcohol (also referred to as "propylene oxide loss"), in the reaction mixture obtained in Comparative Example 2 (added HOAc) were greater than those in Comparative Example 1 (no added HOAc).

### Example 1

The procedure of Comparative Example 2 was repeated, with the proviso that 33 mg of sodium hydroxide (NaOH) was added to the water before injection of the propylene oxide into the reactor. Therefore, the amount of NaOH based on the mixture comprising water and the composition, comprising propylene oxide and carboxylic acid, was 80 ppmw (see Table 1). Reaction of the added NaOH with the HOAc in the crude propylene oxide resulted in formation of (dissolved) sodium acetate (NaOAc) salt.

From Table 2 it appears that the amounts of each of propionaldehyde, 1,3-methylethyldioxalane, and allyl alcohol, and the total amount of propionaldehyde, 1,3-methylethyldioxalane, and allyl alcohol (also referred to as "propylene oxide loss"), in the reaction mixture obtained in Example 1 (added HOAc and base) were smaller than those in Comparative Example 2 (added HOAc but no base) and those in Comparative Example 1 (no added HOAc and no base).

Therefore, these examples show that the propylene oxide loss in Example 1 was only 193 ppmw whereas in Comparative Example 2 it was 1115 ppmw. Therefore, the crude propylene oxide does not first have to be freed from carboxylic acid.

Further, these examples show that by performing the reaction of crude propylene oxide with water upon addition of a base, forming a salt, even less by-products are formed as compared to a case where only propylene oxide and no salt is used. The propylene oxide loss in Example 1 was only 193 ppmw whereas in Comparative Example 1 it was still 470 ppmw.

### Example 2

The procedure of Example 1 was repeated, with the proviso that 66 mg of sodium hydroxide (NaOH) was used, that is to say 160 ppmw based on the mixture comprising water and the composition, comprising propylene oxide and carboxylic acid (see Table 1).

From Table 2 it appears that by increasing the amount of base, the amounts of each of propionaldehyde, 1,3-methylethyldioxalane, and allyl alcohol, and the total amount of propionaldehyde, 1,3-methylethyldioxalane, and allyl alcohol, in the reaction mixture are decreased.

Table 2 also shows that the selectivity to monopropylene glycol is still very high, also in the presence of sodium acetate. These values are higher than those obtained in mixtures with excess sodium hydroxide, as evidenced in US 2,807,651.

**Table 1**

| Example | Carboxylic acid (ppmw; based on PO) | Added NaOH (ppmw; based on PO + water) |
|---|---|---|
| Comp. Ex. 1 | 28 | 0 |
| Comp. Ex. 2 | 1457 | 0 |
| Ex. 1 | 1457 | 80 |
| Ex. 2 | 1457 | 160 |

| | | |
|---|---|---|
| PO = propylene oxide | | |

**Table 2**

| Example | PA (ppmw) | MED (ppmw) | AA (ppmw) | PA, MED and AA (ppmw; "PO loss") | pH^{a} | MPG/DPG (weight ratio) |
|---|---|---|---|---|---|---|
| Comp. Ex. 1 | 170 | 45 | 255 | 470 | 5.2 | 10.7 |
| Comp. Ex. 2 | 485 | 115 | 515 | 1115 | 3.9 | 12.1 |
| Ex. 1 | 6 | 7 | 185 | 193 | 4.8 | 10.7 |
| Ex. 2 | 5 | 2 | 125 | 132 | 5.9 | 9.4 |

| | | | | | | |
|---|---|---|---|---|---|---|
| ^{a} This is the pH of the reaction mixture PA = propionaldehyde, MED = 1,3-methylethyldioxalane, and AA = allyl alcohol | | | | | | |

## Claims

1. Process for the preparation of monopropylene glycol, wherein a base is added to a composition comprising propylene oxide and from 3 to 3000 ppmw of a carboxylic acid, based on total composition, forming a salt, and the propylene oxide is reacted with water in the presence of the salt to obtain monopropylene glycol.

2. Process according to claim 1, wherein water and the composition comprising propylene oxide and carboxylic acid are mixed, and the base is added to the mixture obtained and/or the base is added to said composition and/or to said water before said composition and said water are mixed.

3. Process according to claim 1 or 2, wherein the carboxylic acid comprises acetic acid, formic acid or propionic acid, or a mixture thereof.

4. Process according to any one of claims 1 to 3, wherein the base is an alkali metal, alkaline earth metal or ammonium hydroxide, carbonate or bicarbonate, or ammonia.

5. Process according to claim 4, wherein the base is an alkali metal hydroxide, preferably sodium hydroxide.

6. Process according to any one of claims 1 to 5, wherein the temperature is from 130°C to 350°C.

7. Process according to any one of claims 1 to 6, wherein the weight ratio of water to the composition comprising propylene oxide and carboxylic acid is from 10:1 to 1:1.

8. Monopropylene glycol obtainable according to the process as claimed in any one of claims 1 to 7, that contains allyl alcohol in an amount in the range of from 0.5 to 200 ppmw.
